# DEMANDE DE BREVET EUROPEEN

(11) **EP 4 817 562 A1**
(43) Date de publication de la demande: **07.10.2026**
(21) Numéro de dépôt: 26158446.0
(22) Date de dépôt: 13.02.2026
(51) Int. Cl.: A61F 2/44

(54) **CAGE INTERVERTÉBRALE**

(30) Priorité: 18.02.2025 FR 2501680
(71) Demandeur: Razian, Hassan, 94240 L'Hay-les-Roses (FR)
(72) Inventeur: Razian, Hassan, 94240 L'Hay-les-Roses (FR)
(74) Mandataire: Boüan du Chef du Bos, Louis-Paterne

(57) **Abrégé**

La présente invention concerne les cages intersomatiques.

La cage comporte un élément 10 ayant deux faces 11, 12 ; une lame 31 ayant une demi-lame 31-1, un arbre 48 d'axe 100, la lame étant fixée sur l'arbre de façon que la demi-lame 31-1 soit en déport de l'arbre et que, lors du pivotement de cet arbre 48, la lame 31 prenne toutes positions entre une première position dans laquelle la lame est entièrement contenue dans l'espace entre les plans faciaux 11, 12 et une seconde position dans laquelle la demi-lame 31-1 émerge de cet espace, l'extrémité libre 31-1-1 de la demi-lame comportant une déformation plastique formant ergot 32 en saillie de la demi-lame 31-1, cet ergot étant élastiquement déformé quand **il** est plaqué sur la paroi 15 lorsque la lame 31 est dans sa première position et reprenant sa forme originelle quand la lame est dans sa seconde position au-dessus de la paroi.

## Description

La présente invention concerne les cages intersomatiques aptes à être interposées entre deux vertèbres en remplacement du disque intervertébral originellement situé entre ces deux vertèbres.

Les cages intersomatiques connues comportent notamment : un élément présentant la forme générale d'un disque avec deux faces opposées définissant deux plans faciaux, ces faces opposées étant aptes à venir respectivement au contact des deux vertèbres ; et des moyens qui bloquent l'élément dès l'implantation de la cage entre les deux vertèbres.

Les moyens de blocage connus les plus utilisés sont essentiellement constitués par au moins une lame comprenant au moins une demi-lame, des moyens pour entraîner en rotation cette lame par rapport à l'élément comprenant au moins un arbre de rotation défini selon un axe de rotation, des moyens pour fixer la lame sur l'arbre de rotation de façon que sa demi-lame soit en déport de cet arbre de rotation et de façon que, lors du pivotement de cet arbre de rotation, la lame soit apte à prendre toutes positions entre une première position dans laquelle elle est entièrement contenue dans l'espace défini entre les deux plans faciaux et une seconde position dans laquelle au moins une partie de la demi-lame soit apte à émerger de cet espace pour être apte à s'implanter dans la partie osseuse d'une vertèbre.

Le document WO 2023/216003 A9 illustre ce principe avec une cage expansible où des brins flexibles, portés par un élément mobile pivotant, émergent lors de sa rotation pour s'engager dans les plateaux vertébraux.

Les moyens de blocage décrits ci-dessus donnent de très bons résultats, mais il arrive parfois que, lors de certains mouvements du patient, la lame subisse un retour à sa première position, ce qui n'assure plus le blocage de l'élément par rapport aux vertèbres.

Pour pallier cet inconvénient, ont été prévus des moyens antiretours. Des moyens antiretours connus sont constitués par des butées, comme une vis ou analogue implantée dans l'élément quand la lame est dans sa seconde position, de façon que la vis se positionne en traversant la lame par un orifice prévu à cet effet, ou alors au moins deux vis situées de part et d'autre de la lame pour l'empêcher de subir une rotation inverse à celle qui l'a fait passer de sa première position à sa seconde position.

Mais la solution décrite ci-dessus, même si elle donne de bons résultats, complique le travail du Chirurgien quand il est obligé de reprendre l'implantation d'une telle cage. En effet, du fait de la position de la cage in situ dans le corps du patient, il éprouve alors des difficultés à agir sur ces vis ou analogues pour libérer la lame et retirer la cage.

La présente invention a donc pour but de réaliser une cage intersomatique permettant de pallier grandement les inconvénients des cages intersomatiques antérieures telles que succinctement décrites ci-dessus, tout en facilitant grandement le travail du Chirurgien.

Plus précisément, la présente invention a pour objet de réaliser une cage intersomatique apte à être interposée entre deux vertèbres en remplacement du disque intervertébral originellement situé entre les deux vertèbres, comportant : un élément présentant la forme générale d'un disque avec deux faces opposées définissant deux plans faciaux, ces deux faces étant aptes à venir respectivement au contact des deux vertèbres ; des moyens de blocage dudit élément avec au moins l'une des deux vertèbres, ces moyens étant constitués par au moins une lame comprenant au moins une demi-lame, des moyens pour entraîner en rotation cette dite lame par rapport audit élément comprenant au moins un arbre de rotation défini selon un axe de rotation, des moyens pour fixer cette lame sur ledit arbre de rotation de façon que ladite demi-lame soit en déport de cet arbre de rotation et de façon que, lors du pivotement de cet arbre de rotation, la lame soit apte à prendre toutes positions entre une première position dans laquelle ladite lame est entièrement contenue dans l'espace défini entre les deux plans faciaux et une seconde position dans laquelle au moins une partie de la demi-lame soit apte à émerger de cet espace pour être apte à s'implanter dans la partie osseuse d'un corps vertébral, caractérisée par le fait que l'extrémité libre de ladite demi-lame apte à émerger comporte une partie présentant une déformation plastique pour former un ergot en saillie par rapport au plan général du reste de ladite demi-lame, de façon que cet ergot soit élastiquement déformé quand il est plaqué sur une première portion de paroi de l'élément lorsque ladite lame est située dans sa première position, ledit ergot étant alors sensiblement situé dans le plan général du reste de ladite demi-lame, et de façon qu'il reprenne sa forme originelle quand la lame est dans sa seconde position pour qu'il se positionne au moins partiellement en surplomb au-dessus de ladite première portion de paroi.

D'autres caractéristiques et avantages de l'invention apparaîtront au cours de la description suivante donnée en regard des dessins annexés à titre illustratif mais nullement limitatif, dans lesquels :
La [Fig. 1] représente une vue de dessus schématique partielle d'un mode de réalisation possible de la cage intersomatique selon l'invention, dans laquelle une lame est dite en position « rentrée »,
La [Fig. 2] représente une vue de côté schématique de dessus du mode de réalisation de la cage intersomatique selon la figure 1, dans laquelle la lame est dite en position « sortie/émergente »,
La [Fig. 3] représente une vue schématique de dessus de la partie encadrée référencée EC sur la figure 1, d'un autre mode de réalisation préféré de la cage intersomatique selon l'invention,
La [Fig. 4] représente une vue de dessus schématique correspondant sensiblement à la partie encadrée référencée EC sur la figure 1, d'un autre mode de réalisation avantageux de la cage intersomatique selon l'invention,
La [Fig. 5] représente une vue de côté schématique partielle de dessus d'une autre réalisation de la cage intersomatique selon l'invention partiellement illustrée sur la figure 4, et
La [Fig. 6] représente une vue en perspective d'une ébauche d'un mode de réalisation d'une lame apte à être utilisée dans une cage selon l'invention.

Il est tout d'abord précisé que, lorsque, selon la définition de l'invention, l'objet de l'invention comporte "au moins un" élément ayant une fonction donnée, le mode de réalisation décrit peut comporter plusieurs de ces éléments. Réciproquement, si le mode de réalisation de l'objet selon l'invention tel qu'illustré comporte plusieurs éléments de fonction identique et si, dans la description, il n'est pas spécifié que l'objet selon cette invention doit obligatoirement comporter un nombre particulier de ces éléments, l'objet de l'invention pourra être défini comme comportant "au moins un" de ces éléments.

Il est précisé que lorsque, dans la présente description, une expression définit à elle seule, sans mention particulière spécifique la concernant, un ensemble de caractéristiques structurelles, ces caractéristiques peuvent être prises, pour la définition de l'objet de la protection demandée, quand cela est techniquement possible, soit séparément, soit en combinaison totale et/ou partielle.

Il est de plus précisé que, dans la présente description, si l'adverbe "sensiblement" est associé à un qualificatif d'un moyen donné, ce qualificatif doit être compris au sens strict ou approché.

Il est de plus aussi précisé que, au sens de l'invention, le terme « demi-lame » utilisé dans la présente description est défini, pour plus de compréhension, par rapport aux lames bien connues des cages de l'art antérieur qui sont des lames d'un seul tenant liées à un arbre de rotation en présentant deux portions situées respectivement de part et d'autre de cet arbre de rotation, pour que ces deux portions soient aptes à s'implanter respectivement dans les deux corps vertébraux de deux vertèbres consécutives.

Le terme « demi-lame » utilisé dans la présente description désigne une seule de ces portions de lame qui ne s'implante que dans un seule des deux vertèbres.

La structure d'une telle demi-lame ainsi définie ressort parfaitement des dessins annexés à la présente description.

En outre, pour la meilleure compréhension de la présente invention, il est rappelé ci-dessous les définitions suivantes :
Ergot : petite cheville saillante qui, dans un système mécanique, commande, arrête ou modifie un mouvement.

Déformation élastique : se dit d'un corps qui, lorsque qu'il a été déformé sous l'action d'une force, retrouve sa forme initiale lorsque la force est annulée.

Déformation plastique : se dit d'une déformation permanente d'un corps, même si la force qui l'a déformé est annulée,

Il est cependant rappelé qu'un corps qui a subi une déformation plastique présente malgré tout une possibilité de déformation élastique quand il est soumis à une force, mais de plus faible intensité que celle qui l'a amené dans sa déformation plastique.

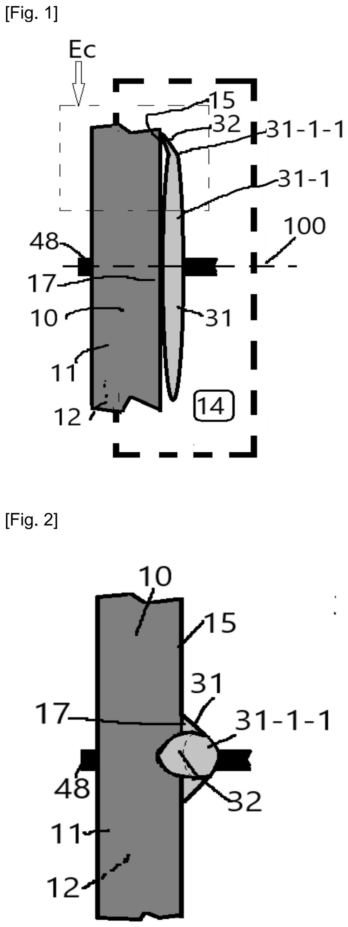

En se référant plus particulièrement aux figures 1 et 2, la cage intersomatique selon l'invention apte à être interposée entre deux vertèbres en remplacement du disque intervertébral originellement situé entre les deux vertèbres comporte un élément 10 présentant la forme générale d'un disque avec deux faces opposées 11, 12 aptes à venir respectivement au contact des deux vertèbres, ces deux faces opposées définissant deux plans faciaux.

Elle comprend des moyens de blocage de cet élément 10 avec au moins l'une des deux vertèbres, ces moyens étant constitués par au moins une lame 31 comprenant au moins une demi-lame 31-1 (selon la définition donnée ci-avant), et des moyens pour entraîner en rotation la lame par rapport à l'élément 10. Ces deniers moyens comprennent schématiquement au moins un arbre de rotation 48 défini selon un axe de rotation 100, des moyens pour fixer la lame 31 sur l'arbre de rotation de façon que la demi-lame 31-1 soit en déport de cet arbre de rotation et de façon que, lors du pivotement de l'arbre de rotation 48, la lame 31 soit apte à prendre toutes positions entre une première position dans laquelle elle est entièrement contenue dans l'espace défini entre les deux plans faciaux 11, 12 et une seconde position dans laquelle au moins une partie de la demi-lame 31-1 de la lame soit apte à émerger de cet espace pour être apte à s'implanter dans la partie osseuse d'un corps vertébral.

Selon une caractéristique selon l'invention, l'extrémité libre 31-1-1 de la demi-lame 31-1 apte à émerger comporte une partie présentant une déformation plastique pour former un ergot 32 (selon la définition donnée ci-avant) en saillie par rapport au plan général du reste de la demi-lame 31-1, de façon que cet ergot soit élastiquement déformé quand il est plaqué sur une première portion de paroi 15 de l'élément 10 lorsque la lame 31 est située dans sa première position, l'ergot étant alors sensiblement situé dans le plan général du reste de la demi-lame 31-1, et de façon qu'il reprenne sa forme originelle quand la lame est dans sa seconde position pour qu'il se positionne au moins partiellement en surplomb au-dessus de la première portion de paroi 15.

Selon un premier mode de réalisation de la cage schématiquement illustré sur les figures 1-3, cet ergot 32 est constitué par un pli de l'extrémité libre 31-1-1 de la demi-lame 31-1. Cette réalisation permet d'arriver au but que se propose d'atteindre l'invention, mais présente malgré tout l'inconvénient constitué par le fait que la demi-lame 31-1 ne s'implante pas d'une grande quantité, ce qui en limite son utilisation essentiellement en fonction des configurations actuelles des éléments 10 qui, comme mentionné ci-avant, se présentent sous une forme qui peut être comparée sensiblement celle d'un disque.

De plus : les éléments 10 sont en majorité réalisés en un matériau plastique, par exemple du Peek (polyétheréthercétone), mais aussi en Titane, TA6V-ELI ou analogue.

Quant à la lame 31, elle est au moins en l'une des matières suivantes : Titane, TA6V-ELI, acier inoxydable, céramique ou analogue.

En conséquence, lorsque la lame 31, et donc la demi-lame 31-1, passe de sa première position à sa seconde position, l'ergot 32 bandé frotte sur la paroi 15 de l'élément 10 qui est en un matériau relativement plus tendre que cet ergot, et ce dernier peut donc griffer la paroi 15, ce qui risque d'entraîner une détérioration relativement rapide de l'élément 10.

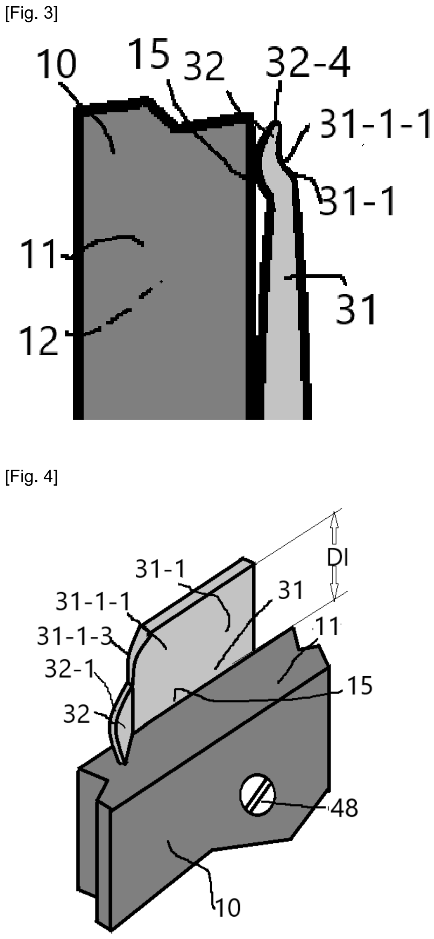

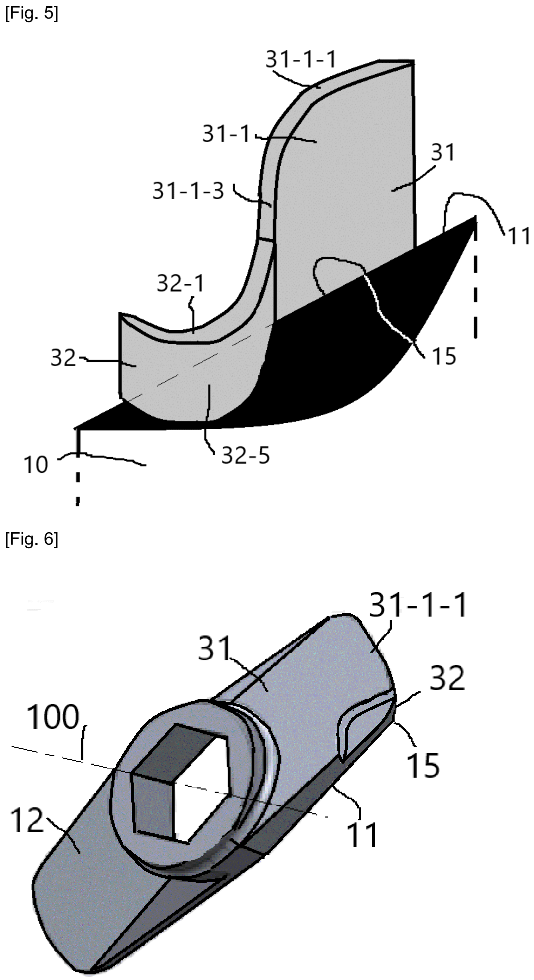

Pour tenter de pallier cet inconvénient, selon un autre mode de réalisation, l'ergot 32 présente avantageusement une configuration comme celle illustrée sur les figures 3 et 5.

Dans ce mode de réalisation, la partie de l'ergot 32 qui est apte à être plaquée sur la première portion de paroi 15 présente une portion de surface courbe non anguleuse 32-5 dont l'extrémité libre 32-4 est sensiblement en forme de biseau 32-4. La pointe du biseau étant éloignée de cette première portion de paroi 15 ne peut donc pas frotter sur cette paroi.

Pour répondre aux configurations actuelles de l'élément 10, d'une part la partie de la lame 31 se situant au niveau de l'arbre de rotation 48 est plaquée en force contre une seconde portion de paroi 17 de l'élément 10 et d'autre part la première portion de paroi 15 et la seconde portion de paroi 17 sont définies dans un même plan et appartiennent à une même paroi.

Selon la configuration actuelle préférentielle de l'élément 10, à savoir celle où il comporte une percée centrale traversante 14, la lame étant située en totalité dans cette percée quand elle est dans première position, et partiellement émergente quand elle est dans sa seconde position, la même paroi définie ci-avant est une paroi de la percée traversante 14, comme évoqué schématiquement sur la figure 1.

Dans ce mode de configuration de l'élément 10, l'ergot 32 est très avantageusement situé sur un bord latéral 31-1-3 de l'extrémité libre 31-1-1 de la demi-lame 31-1, figures 4 et 5.

Encore plus préférentiellement : le bord 32-1 de l'ergot 32 le plus proche du sommet de l'extrémité libre 31-1-1 permet que la demi-lame 31-1 peut s'implanter dans l'os d'une vertèbre d'une quantité relativement importante qui peut être définie sur la distance DI entre le bord de l'ergot 32 qui est sur le sommet de la paroi 15 et le sommet 31-1-1 de la demi-lame 31-1 comme illustré sur la figure 4.

La cage selon l'invention s'utilise comme décrit ci-après.

La cage est préparée originellement avant son implantation de façon que la lame 31 soit dans sa première position, avec l'ergot 32 bandé, sa partie courbe 32-5 étant plaquée contre la paroi 15 de la cavité 14.

Pour son implantation, de façon connue, le Chirurgien l'introduit entre deux vertèbres en remplacement du disque intervertébral originellement situé entre ces deux vertèbres.

Après la mise en place de la cage, le Chirurgien fait pivoter la lame 31 pour que la demi-lame 31-1 vienne s'implanter dans une partie osseuse d'une vertèbre. En début de ce mouvement de rotation, l'ergot 32 frotte sur la paroi 15 par sa portion courbe 32-5, donc sans endommager la surface de cette paroi 15. Après une certaine amplitude de rotation et jusqu'à la fin de la rotation de la lame, l'ergot se détend et se positionne en surplomb au-dessus de la paroi 15.

Dans cette position, l'ergot 32 empêche tout retour en arrière de la lame 31, figures 2, 4 et 5. Ce retour ne pourra se faire, en cas de besoin, que par une action délibérée du Chirurgien qui appliquera alors sur la lame, pour la faire pivoter, une force relativement élevée, pour faire subir à l'ergot, par rapport à l'élément 10, une déformation, mais uniquement élastique, de façon qu'il reprenne sa position évoquée sur les figures 1 et 3.

A cette description, il apparaît que l'invention pallie vraiment l'inconvénient des butées amovibles comme rappelé au préambule de la présente description et simplifie grandement le travail du Chirurgien aussi bien lors de la pose de la cage intersomatique que pour une opération de reprise.

L'ergot 32 peut être obtenu de différentes façons : par exemple une surépaisseur de la lame 31, ou un enfoncement borgne (figure 6) avec dépassement d'au moins un côté de la lame 31, ou une combinaison de ces deux possibilités, ou analogues.

## Revendications

1. Cage intersomatique apte à être interposée entre deux vertèbres en remplacement du disque intervertébral originellement situé entre les deux vertèbres, comportant : un élément (10) présentant la forme générale d'un disque avec deux faces opposées (11, 12) définissant deux plans faciaux, ces deux faces étant aptes à venir respectivement au contact des deux vertèbres ; des moyens de blocage dudit élément (10) avec au moins l'une des deux vertèbres, ces moyens étant constitués par au moins une lame (31) comprenant au moins une demi-lame (31-1), des moyens pour entraîner en rotation cette dite lame par rapport audit élément (10) comprenant au moins un arbre de rotation (48) défini selon un axe de rotation (100), des moyens pour fixer cette lame (31) sur ledit arbre de rotation de façon que ladite demi-lame (31-1) soit en déport de cet arbre de rotation et de façon que, lors du pivotement de cet arbre de rotation (48), la lame (31) soit apte à prendre toutes positions entre une première position dans laquelle elle est entièrement contenue dans l'espace défini entre les deux plans faciaux (11, 12) et une seconde position dans laquelle au moins une partie de la demi-lame (31-1) soit apte à émerger de cet espace pour être apte à s'implanter dans la partie osseuse d'un corps vertébral, **caractérisée par le fait que** l'extrémité libre (31-1-1) de ladite demi-lame (31-1) apte à émerger comporte une partie présentant une déformation plastique pour former un ergot (32) en saillie par rapport au plan général du reste de ladite demi-lame (31-1), de façon que cet ergot soit élastiquement déformé quand il est plaqué sur une première portion de paroi (15) de l'élément (10) lorsque ladite lame (31) est située dans sa première position, ledit ergot étant alors sensiblement situé dans le plan général du reste de ladite demi-lame (31-1), et de façon qu'il reprenne sa forme originelle quand la lame est dans sa seconde position pour qu'il se positionne au moins partiellement en surplomb au-dessus de ladite première portion de paroi (15).

2. Cage selon la revendication 1, **caractérisée par le fait que** ledit élément (10) est en un matériau plastique.

3. Cage selon l'une des revendications 1 et 2, **caractérisée par le fait que** ledit ergot (32) est apte à être plaqué sur la première portion de paroi (15) par une portion de surface courbe non anguleuse (32-5), cette portion de surface courbe (32-5) se terminant, à son extrémité libre (32-4), sensiblement en forme de biseau (32-4), la pointe du dit biseau étant éloignée de cette dite première portion de paroi (15).

4. Cage selon l'une des revendications précédentes, **caractérisée par le fait que** la partie de ladite lame (31) se situant au niveau dudit arbre de rotation (48) est plaquée en force contre une seconde portion de paroi (17) dudit élément (10).

5. Cage selon la revendication 4, **caractérisée par le fait que** ladite première portion de paroi (15) et ladite seconde portion de paroi (17) appartiennent à une même paroi définie dans un plan.

6. Cage selon la revendication 5, **caractérisée par le fait que** ledit élément (10) comporte une percée centrale traversante (14), ladite même paroi étant une paroi de la percée traversante (14).

7. Cage selon l'une des revendications précédentes, **caractérisée par le fait que** ledit ergot (32) est situé sur un bord latéral (31-1-3) de l'extrémité libre (31-1-1) de ladite demi-lame (31-1).

8. Cage selon la revendication 7, **caractérisée par le fait que** le bord (32-1) dudit ergot (32) le plus proche du sommet de l'extrémité libre (31-1-1) de ladite demi-lame (31-1) est à distance non nulle de ce sommet.

9. Cage selon l'une des revendications précédentes, **caractérisée par le fait que** ladite lame est en l'une des matières suivantes : titane, acier inoxydable, céramique.

10. Cage selon l'une des revendications précédentes, **caractérisée par le fait que** ledit ergot (32) est constitué selon l'une des possibilités suivantes : surépaisseur de la lame (31), enfoncement borgne avec des dépassements d'au moins un côté de ladite lame, une combinaison de ces deux possibilités.
